# EUROPEAN PATENT APPLICATION

(11) **EP 4 317 461 A1**
(43) Date of publication of application: **07.02.2024**
(21) Application number: 22780522.3
(22) Date of filing: 24.03.2022
(51) Int. Cl.: C12R 1/125, C12N 1/21, C12N 15/09, C12N 15/31, C12N 15/53, C12P 21/02, C07K 14/32

(54) **CYCLIC LIPOPEPTIDE-PRODUCING MICROBIAL STRAIN AND METHOD FOR PRODUCING CYCLIC LIPOPEPTIDE**

(30) Priority: 29.03.2021 JP 2021056115
(71) Applicant: Kaneka Corporation, Osaka-shi, Osaka 530-8288 (JP)
(72) Inventor: AOKI Rina, Takasago-shi, Hyogo 676-8688 (JP); KOBAYASHI Shingo, Takasago-shi, Hyogo 676-8688 (JP); TAOKA Naoaki, Tokyo 107-6028 (JP)
(74) Representative: Hoffmann Eitle
(86) International application number: PCT/JP2022/014183
(87) International publication number: WO 2022/210308

(57) **Abstract**

This invention is intended to improve productivity of a cyclic lipopeptide, such as surfactin, via microbial culture. This invention provides a cyclic lipopeptide-producing microbial strain, lacking at least one of a gene encoding betaine aldehyde dehydrogenase (EC: 1.2.1.8) or a gene encoding choline dehydrogenase (EC: 1.1.1.1) and a method for producing a cyclic lipopeptide comprising culturing such microbial strain.

## Description

### Technical Field

The present invention relates to a cyclic lipopeptide-producing microbial strain and a method for producing a cyclic lipopeptide using such microbial strain.

### Background Art

Cyclic lipopeptides represented by surfactin and iturin are amphiphilic substances derived from microorganisms. For example, surfactin has been extensively used as a highly safe and biodegradable biosurfactant for pharmaceuticals, cosmetics, and food products. In addition, such cyclic lipopeptides exert not only surfactant activity but also exert excellent antibacterial or antifungal activity on an extensive range of bacteria or fungi. Accordingly, application of cyclic lipopeptides is expected in an extensive range of fields, including medicine, food manufacture, agriculture, and environmental health, as, for example, antibacterial agents, fungicides, therapeutic agents for infectious diseases, or plant-disease control agents.

Cyclic lipopeptides, such as surfactin and iturin, are produced by microorganisms of the genus *Bacillus,* and, accordingly, industrial production of cyclic lipopeptides is performed by culture of microorganisms of the genus *Bacillus* (e.g., Patent Document 1). Accordingly, productivity thereof would be critical in industrial production of useful substances mediated by such microorganisms.

To date, various methods have been examined to improve surfactin productivity via culture of microorganisms of the genus *Bacillus;* however, many of such methods would require the addition of special components to a medium or strict control of culture conditions and, accordingly, such methods were not sufficient in terms of productivity or cost-effectiveness.

Several *Bacillus subtilis* mutant strains exhibiting high surfactin productivity have also been reported. For example, Patent Document 2 discloses the *Bacillus subtilis* ATCC55033 strain that can produce surfactin at high concentration by subjecting the *Bacillus subtilis* ATCC21332 strain to mutagenesis with NMG. Non-Patent Document 1 reports that a mutant strain derived from the *Bacillus subtilis* ATCC21332 strain by ultraviolet application exhibits surfactin productivity that is at least 3 times greater than that of the parent strain and that such mutation is located between argC4 and hisA1 on the genetic map. However, such mutant strains are not microbial strains resulting from modification of particular target genes associated with the mutations thereof, and, accordingly, such mutant strain cannot be stably supplied in a large quantity for industrial production.

### Prior Art Documents

### Patent Documents

Patent Document 1: JP Patent No. 3,635,638
Patent Document 2: JP Patent No. 3,030,789

### Non-Patent Documents

Non-Patent Document 1: Appl. Microbiol. Biotech., 31: 486-489, 1989

### Summary of the Invention

### Objects to Be Attained by the Invention

Accordingly, an object of the present invention is to provide a microbial strain that is excellent in productivity of cyclic lipopeptides, such as surfactin, and can be stably supplied in a large quantity for industrial production of cyclic lipopeptides, and another object is to improve productivity of cyclic lipopeptides by microbial culture.

### Means for Attaining the objects

The present inventors have conducted concentrated studies in order to attain the above objects. As a result, they discovered that a microbial strain having disruption of at least either one of the genes associated with the biosynthetic pathway of glycine betaine, which is an osmoregulatory substance in a cell; i.e., a gene encoding betaine aldehyde dehydrogenase (EC: 1.2.1.8) (the gbsA gene) or a gene encoding choline dehydrogenase (EC: 1.1.1.1) (the gbsB gene), would exhibit significantly improved surfactin productivity. This has led to the completion of the present invention.

Specifically, the present invention includes the following.
[1] A cyclic lipopeptide-producing microbial strain, having disruption of at least either one of the gene (1) or (2):
   (1) a gene encoding betaine aldehyde dehydrogenase (EC: 1.2.1.8); or
   (2) a gene encoding choline dehydrogenase (EC: 1.1.1.1).
[2] The microbial strain according to [1], which is a genetically modified microbial strain whose host is a bacterium.
[3] The microbial strain according to [2], wherein the bacterium is a Gram-positive bacterium.
[4] The microbial strain according to [3], wherein the Gram-positive bacterium is a bacterium of the genus *Bacillus.*
[5] The microbial strain according to [4], wherein the bacterium of the genus *Bacillus* is *Bacillus subtilis.*
[6] The microbial strain according to any of [1] to [5], wherein the cyclic lipopeptide is at least one cyclic lipopeptide selected from among a surfactin-family cyclic lipopeptide, an iturin-family cyclic lipopeptide, and a fengycin-family cyclic lipopeptide.
[7] The microbial strain according to any of [1] to [6], wherein the cyclic lipopeptide is surfactin.
[8] A method for producing a cyclic lipopeptide comprising culturing the microbial strain according to any of [1] to [7] in a medium.
[9] The method for producing a cyclic lipopeptide according to [8], wherein the medium contains grounded beans or an extract thereof.
[10] The method for producing a cyclic lipopeptide according to [9], wherein the beans are soybeans.

This patent application claims priority from Japanese Patent Application No. 2021-56115 filed on March 29, 2021, and it includes part or all of the contents as disclosed in the description thereof.

### Effects of the Invention

The present invention provides a microbial strain exhibiting cyclic lipopeptide productivity superior to that of a wild-type strain or a known mutant strain. With the use of such microbial strain, productivity of industrially applicable cyclic lipopeptides can be improved.

### Embodiments of the Invention

### <Host microorganisms>

Microorganisms serving as host strains (parent strains) of the microbial strains having disruption of at least either one of (1) a gene encoding betaine aldehyde dehydrogenase (EC: 1.2.1.8) (gene name: gbsA) or (2) a gene encoding choline dehydrogenase (EC: 1.1.1.1) (gene name: gbsB) according to one or more embodiments of the present invention are preferably bacteria, more preferably Gram-positive bacteria, further preferably bacteria of the genus *Bacillus,* the genus *Paenibacillus,* the genus *Brevibacillus,* the genus *Tumebacillus,* and the genus *Streptomyces,* furthermore preferably bacteria of the genus *Bacillus,* particularly preferably *Bacillus subtilis, Bacillus velezensis, Bacillus amyloliquefaciens, Bacillus siamensis, Bacillus atrophaeus, Bacillus vallismortis, Bacillus sonorensis, Bacillus halotolerans, Bacillus anthracis, Bacillus cereus, Bacillus thuringiensis, Bacillus mycoides, Bacillus licheniformis, Bacillus paralicheniformis, Bacillus swezeyi, Bacillus genomospecies,* and *Bacillus methylotrophicus,* and the most preferably *Bacillus subtilis.* Microorganisms serving as host strains (parent strains) may be wild-type strains or mutant strains.

The microbial strain according to one or more embodiments of the present invention is a transformant (genetically modified microorganism) derived from its host by disruption of at least either one gene of betaine aldehyde dehydrogenase (EC:1.2.1.8) or choline dehydrogenase (EC: 1.1.1.1).

### <Betaine aldehyde dehydrogenase (EC:1.2.1.8)>

Betaine that regulates the osmotic pressure in cells of many bacteria, plants and animals is known to be synthesized in several pathways. One of such pathways is a two-steps synthetic pathway comprising (i) conversion from choline to betaine aldehyde and (ii) conversion from betaine aldehyde to betaine. Betaine aldehyde dehydrogenase (EC: 1.2.1.8) is an enzyme that catalyzes the second-step reaction described above and it converts glycine betaine aldehyde into glycine betaine using NAD+ as a coenzyme.

The nucleotide sequence of betaine aldehyde dehydrogenase derived from *Bacillus subtilis* as an example of betaine aldehyde dehydrogenase and the amino acid sequence encoded by such nucleotide sequence are shown in SEQ ID NO: 1 and SEQ ID NO: 2, respectively.

Betaine aldehyde dehydrogenase is not limited to the betaine aldehyde dehydrogenase consisting of the amino acid sequence as shown in SEQ ID NO: 2, and other polypeptides having betaine aldehyde dehydrogenase activity, such as active mutants of the betaine aldehyde dehydrogenase or orthologs of different species, may be used. Other polypeptides having betaine aldehyde dehydrogenase activity preferably exhibit activity of 10% or higher, more preferably 40% or higher, more preferably 60% or higher, more preferably 80% or higher, and further preferably 90% or higher, compared with the activity of the betaine aldehyde dehydrogenase consisting of the amino acid sequence as shown in SEQ ID NO: 2.

Specific examples of betaine aldehyde dehydrogenase include:
(1A) a polypeptide consisting of the amino acid sequence as shown in SEQ ID NO: 2;
(1B) a polypeptide consisting of an amino acid sequence derived from the amino acid sequence as shown in SEQ ID NO: 2 by addition, deletion, or substitution of 1 or a plurality of amino acids (particularly preferably, a polypeptide consisting of an amino acid sequence derived from the amino acid sequence as shown in SEQ ID NO: 2 by substitution, deletion, and/or addition, and preferably by deletion and/or addition, of 1 or a plurality of amino acids at either or both of the N terminus and the C terminus) and having betaine aldehyde dehydrogenase activity;
(1C) a polypeptide consisting of an amino acid sequence having 80% or higher, preferably 85% or higher, and more preferably 90% or higher, 95% or higher, 97% or higher, 98% or higher, or 99% or higher sequence identity to the amino acid sequence as shown in SEQ ID NO: 2 and having betaine aldehyde dehydrogenase activity; and
(1D) a fragment of any of the polypeptides (1A) to (1C) having betaine aldehyde dehydrogenase activity.

In (1B) above, the term "a plurality of" refers to, for example, 2 to 20, 2 to 15, 2 to 10, 2 to 7, 2 to 5, 2 to 4, or 2 or 3. Amino acid substitution is preferably conservative amino acid substitution. The term "conservative amino acid substitution" refers to substitution between amino acids having similar properties in terms of, for example, electric charge, side chains, polarity, and aromaticity. Amino acids having similar properties can be classified into: for example, basic amino acids (arginine, lysine, and histidine), acidic amino acids (aspartic acid and glutamic acid), uncharged polar amino acids (glycine, asparagine, glutamine, serine, threonine, cysteine, and tyrosine), nonpolar amino acids (leucine, isoleucine, alanine, valine, proline, phenylalanine, tryptophan, and methionine), branched-chain amino acids (leucine, valine, and isoleucine), and aromatic amino acids (phenylalanine, tyrosine, tryptophan, and histidine), or the like. Hereafter, the term "conservative amino acid substitution" is used in the same sense.

In (1C) above, the "sequence identity" is a value determined by aligning 2 amino acid sequences, introducing gaps, according to need, so as to maximize the extent of amino acid consistency therebetween, and determining a percentage (%) of identical amino acids based on the total number of amino acids constituting the protein as shown in SEQ ID NO: 2. The "sequence identity" can be determined with the use of protein search systems, such as BLAST or FASTA (Karlin, S. et al., 1993, Proc. Natl. Acad. Sci., U.S.A., 90: 5873-5877; Altschul, S. F. et al., 1990, J. Mol. Biol., 215: 403-410; Pearson, W. R. et al., 1988, Proc. Natl. Acad. Sci., U.S.A., 85: 2444-2448). Hereafter, the term "sequence identity" of amino acid sequences is used in the same sense.

The fragment (1D) can be a polypeptide comprising preferably 200 or more, more preferably 300 or more, and further preferably 400 or more amino acids.

The term "a gene encoding betaine aldehyde dehydrogenase (EC: 1.2.1.8)" refers to a nucleic acid (DNA or RNA, preferably, DNA) encoding the amino acid sequence of betaine aldehyde dehydrogenase, and such gene is referred to as "gbsA." The gbsA gene is included in the genome DNA in the chromosome of the wild-type microorganism before disruption of betaine aldehyde dehydrogenase therein.

SEQ ID NO: 1 shows an example of DNA encoding the amino acid sequence of betaine aldehyde dehydrogenase derived from *Bacillus subtilis* as shown in SEQ ID NO: 2. The nucleotide sequence of the nucleic acid encoding the amino acid sequence of betaine aldehyde dehydrogenase may be codon-optimized for the host. It should be noted that the nucleotide sequence as shown in SEQ ID NO: 1 is not always present in that state in the genome DNA of the microbial strain. The nucleotide sequence as shown in SEQ ID NO: 1 may be an exon sequence comprising one or more intron sequences therein.

Specific examples of nucleotide sequences of genes encoding the amino acid sequence of betaine aldehyde dehydrogenase include:
(1E) the nucleotide sequence as shown in SEQ ID NO: 1;
(1F) a nucleotide sequence derived from the nucleotide sequence as shown in SEQ ID NO: 1 by addition, deletion, or substitution of 1 or a plurality of nucleotides (particularly preferably, a nucleotide sequence derived from the nucleotide sequence as shown in SEQ ID NO: 1 by substitution, deletion, and/or addition, and preferably by deletion and/or addition, of 1 or a plurality of nucleotides at either or both of the 5' terminus and the 3' terminus) and encoding a polypeptide having betaine aldehyde dehydrogenase activity;
(1G) a nucleotide sequence having 80% or higher, preferably 85% or higher, and more preferably 90% or higher, 95% or higher, 97% or higher, 98% or higher, or 99% or higher sequence identity to the nucleotide sequence as shown in SEQ ID NO: 1 and encoding a polypeptide having betaine aldehyde dehydrogenase activity;
(1H) a partial nucleotide sequence of any of the nucleotide sequences (1E) to (1G) encoding an amino acid sequence of a polypeptide having betaine aldehyde dehydrogenase activity;
(1I) a nucleotide sequence derived from any of the nucleotide sequences (1E) to (1H) by introduction of silent mutation (which is nucleotide substitution that does not alter amino acids to encode);
(1J) a nucleotide sequence encoding the amino acid sequence of any of the polypeptides (1A) to (1D); and
(1K) a nucleotide sequence comprising, as an exon sequence, any of the nucleotide sequences (1E) to (1J) and one or more intron sequences therein.

In (1G) above, the "sequence identity" is a value determined by aligning 2 nucleotide sequences, introducing gaps, according to need, so as to maximize the extent of nucleotide consistency therebetween, and determining a percentage (%) of identical nucleotides based on the total number of nucleotides in the nucleotide sequence as shown in SEQ ID NO: 1. The "sequence identity" can be determined with the use of nucleotide sequence search systems, such as BLAST or FASTA (Karlin, S. et al., 1993, Proc. Natl. Acad. Sci., U.S.A., 90: 5873-5877; Altschul, S. F. et al., 1990, J. Mol. Biol., 215: 403-410; Pearson, W. R. et al., 1988, Proc. Natl. Acad. Sci., U.S.A., 85: 2444-2448). Hereafter, the "sequence identity" of nucleotide sequences is used in the same sense.

In (1F) above, the term "a plurality of" refers to, for example, 2 to 60, 2 to 45, 2 to 30, 2 to 21, 2 to 15, 2 to 6, or 2 or 3.

### <Choline dehydrogenase (EC:1.1.1.1)>

Choline dehydrogenase (EC: 1.1.1.1) is an enzyme that catalyzes the first-step reaction of the glycine betaine synthetic pathway and it converts choline into glycine betaine aldehyde using NAD+ as a coenzyme.

The nucleotide sequence of choline dehydrogenase derived from *Bacillus subtilis* as an example of choline dehydrogenase and the amino acid sequence encoded by such nucleotide sequence are shown in SEQ ID NO: 3 and SEQ ID NO: 4, respectively.

Choline dehydrogenase is not limited to the choline dehydrogenase consisting of the amino acid sequence as shown in SEQ ID NO: 4, and other polypeptides having choline dehydrogenase activity, such as active mutants of the choline dehydrogenase or orthologs of different species, may be used. Other polypeptides having choline dehydrogenase activity preferably exhibit activity of 10% or higher, more preferably 40% or higher, more preferably 60% or higher, more preferably 80% or higher, and further preferably 90% or higher, compared with the activity of the choline dehydrogenase consisting of the amino acid sequence as shown in SEQ ID NO: 4.

Specific examples of choline dehydrogenase include:
(2A) a polypeptide consisting of the amino acid sequence as shown in SEQ ID NO: 4;
(2B) a polypeptide consisting of an amino acid sequence derived from the amino acid sequence as shown in SEQ ID NO: 4 by addition, deletion, or substitution of 1 or a plurality of amino acids (particularly preferably, a polypeptide consisting of an amino acid sequence derived from the amino acid sequence as shown in SEQ ID NO: 4 by substitution, deletion, and/or addition, and preferably by deletion and/or addition, of 1 or a plurality of amino acids at either or both of the N terminus and the C terminus) and having choline dehydrogenase activity;
(2C) a polypeptide consisting of an amino acid sequence having 80% or higher, preferably 85% or higher, and more preferably 90% or higher, 95% or higher, 97% or higher, 98% or higher, or 99% or higher sequence identity to the amino acid sequence as shown in SEQ ID NO: 4 and having choline dehydrogenase activity; and
(2D) a fragment of any of the polypeptides (2A) to (2C) having choline dehydrogenase activity.

In (1B) above, the term "a plurality of" refers to, for example, 2 to 20, 2 to 15, 2 to 10, 2 to 7, 2 to 5, 2 to 4, or 2 or 3. Amino acid substitution is preferably conservative amino acid substitution.

In (2C) above, the "sequence identity" is a value determined by aligning 2 amino acid sequences, introducing gaps, according to need, so as to maximize the extent of amino acid consistency therebetween, and determining a percentage (%) of identical amino acids based on the total number of amino acids constituting the protein as shown in SEQ ID NO: 4.

The fragment (2D) can be a polypeptide comprising preferably 200 or more, more preferably 300 or more, and further preferably 400 or more amino acids.

The term "a gene encoding choline dehydrogenase" refers to a nucleic acid (DNA or RNA, preferably DNA) encoding the amino acid sequence of choline dehydrogenase, and such gene is referred to as "gbsB." The gbsB gene is included in the genome DNA in the chromosome of the wild-type microorganism before disruption of choline dehydrogenase therein.

SEQ ID NO: 3 shows an example of DNA encoding the amino acid sequence of choline dehydrogenase derived from *Bacillus subtilis* as shown in SEQ ID NO: 4. The nucleotide sequence of the nucleic acid encoding the amino acid sequence of choline dehydrogenase may be codon-optimized for the host. It should be noted that the nucleotide sequence as shown in SEQ ID NO: 3 is not always present in that state in the genome DNA of the microbial strain. The nucleotide sequence as shown in SEQ ID NO: 3 may be an exon sequence comprising one or more intron sequences therein.

Specific examples of nucleotide sequences of genes encoding the amino acid sequence of choline dehydrogenase include:
(2E) the nucleotide sequence as shown in SEQ ID NO: 3;
(2F) a nucleotide sequence derived from the nucleotide sequence as shown in SEQ ID NO: 3 by addition, deletion, or substitution of 1 or a plurality of nucleotides (particularly preferably, a nucleotide sequence derived from the nucleotide sequence as shown in SEQ ID NO: 3 by substitution, deletion, and/or addition, and preferably by deletion and/or addition, of 1 or a plurality of nucleotides at either or both of the 5' terminus and the 3' terminus) and encoding a polypeptide having choline dehydrogenase activity;
(2G) a nucleotide sequence having 80% or higher, preferably 85% or higher, and more preferably 90% or higher, 95% or higher, 97% or higher, 98% or higher, or 99% or higher sequence identity to the nucleotide sequence as shown in SEQ ID NO: 3 and encoding a polypeptide having choline dehydrogenase activity;
(2H) a partial nucleotide sequence of any of the nucleotide sequences (2E) to (2G) encoding an amino acid sequence of a polypeptide having choline dehydrogenase activity;
(2I) a nucleotide sequence derived from any of the nucleotide sequences (2E) to (2H) by introduction of silent mutation (which is nucleotide substitution that does not alter amino acids to encode);
(2J) a nucleotide sequence encoding the amino acid sequence of any of the polypeptides (2A) to (2D); and
(2K) a nucleotide sequence comprising, as an exon sequence, any of the nucleotide sequences (2E) to (2J) and one or more intron sequences therein.

In (2F) above, the term "a plurality of" refers to, for example, 2 to 60, 2 to 45, 2 to 30, 2 to 21, 2 to 15, 2 to 6, or 2 or 3.

### < Microbial strain of the present invention>

The microbial strain according to one or more embodiments of the present invention is a microbial strain having disruption of at least either one of (1) a gene encoding betaine aldehyde dehydrogenase (EC:1.2.1.8) (the gbsA gene) or (2) a gene encoding choline dehydrogenase (EC: 1.1.1.1) (the gbsB gene). Gene disruption may be disruption of either of the gbsA gene or the gbsB gene or both thereof.

When the gbsA gene and the gbsB gene to be disrupted (these genes may be referred to as "gene to be disrupted") is "disrupted" in the present invention, activity of a protein encoded by the gene to be disrupted is lowered, compared with the activity of the host strain, or the activity is completely missing. The microbial strain according to one or more embodiments of the present invention is deprived of functions of the gene to be disrupted, or such functions are lowered in the microbial strain. In such microbial strain, specifically, the expression level of mRNA, which is a transcription product, or a protein, which is a translation product, of the gene to be disrupted is lowered or nearly zero, mRNA, which is a transcription product, or a protein, which is a translation product, of the gene to be disrupted does not normally function as mRNA or a protein, or mRNA, which is a transcription product, or a protein, which is a translation product, of the gene to be disrupted is not generated and thus does not completely function as mRNA or a protein.

Disruption of the gene to be disrupted can be achieved by, for example, artificial modification of the gene of the host strain. Such modification can be achieved by, for example, mutagenesis, genetic modification technique, or method for suppressing gene expression.

Mutagenesis can be performed via application of ultraviolet rays, application of radiation (e.g., γ rays), or via treatment with a common agent causing mutation, such as N-methyl-N'-nitro-N-nitrosoguanidine (MNNG), ethyl methanesulfonate (EMS), or methyl methane sulfonate (MMS).

Genetic modification technique can be performed in accordance with a known technique (e.g., FEMS Microbiology Letters 165, 1998, 335-340, JOURNAL OF BACTERIOLOGY, Dec. 1995, pp. 7171-7177, Curr. Genet., 1986, 10 (8): pp. 573-578, or WO 98/14600).

Examples of method for suppressing gene expression include methods involving the use of an RNAi inducible nucleic acid exerting RNA interference activity on mRNA of the gene to be deleted (e.g., siRNA, shRNA, and dsRNA), a nucleic acid suppressing translation of mRNA of the gene to be deleted (e.g., antisense nucleic acid, miRNA, and ribozyme nucleic acid), and a nucleic acid suppressing transcription of the gene to be deleted (e.g., decoy nucleic acid). The term "RNAi inducible nucleic acid" refers to a double-stranded RNA molecule that can induce RNA interference when it is introduced into a cell. The term "RNA interference" refers to an effect of a double-stranded RNA comprising a nucleotide sequence identical to that of mRNA (or a partial sequence thereof) to suppress expression of the mRNA. Examples of RNAi inducible nucleic acids include siRNA and shRNA comprising a stem-loop structure in a part thereof (small hairpin RNA). From the viewpoint of the strength for suppressing transcription activity, siRNA is preferable. Specifically, siRNA consists of a sense strand comprising a nucleotide sequence in mRNA corresponding to the nucleotide sequence as shown in SEQ ID NO: 1 or 3 and an antisense strand comprising a sequence complementary to the sense strand. While an siRNA length is not particularly limited as long as RNA interference can be induced, in general, siRNA comprises approximately 18 to 25 nucleotides. siRNA that acts on the gene to be deleted may comprise approximately 1 to 5 additional nucleotides at the 5'- or 3'- terminus of either or both the sense strand and the antisense strand.

Another example of method for suppressing gene expression is the CRISPRi (CRISPR interference) method for suppressing transcription by recruiting the Cas9 protein without endonuclease activity (dCas9) with the aid of gRNA (or crRNA or tracrRNA) to a promoter region or a transcription initiation region of the gene to be deleted. Methods for designing gRNA and crRNA are well known, and gRNA or crRNA may be designed, so that a region of approximately 20-mer at the 5' terminus thereof can hybridize under physiological conditions to the target sequence.

Disruption of the gene to be disrupted is more preferably disruption of the gene to be disrupted in the genome DNA of the microbial strain. The disruption of the gene to be disrupted may be disruption of a part or the whole of the expression regulatory sequence or disruption of a part or the whole of the coding region of the amino acid sequence of the protein. The term "disruption" used herein refers to deletion or damage, preferably, deletion.

The entire gene, including upstream and downstream sequences of the gene to be disrupted, may be deleted in the genome DNA of the host strain. When a part or the whole of the coding region of the amino acid sequence of the protein encoded by the gene to be disrupted is to be deleted, the coding region in either of the N-terminal region, the internal region, the C-terminal region, and other regions may be deleted, provided that protein activity can be lowered. In general, the gene can be inactivated with certainty by deletion of a longer region. The reading frames of the upstream and downstream sequences of the region to be deleted are preferably inconsistent. A preferable embodiment is directed to a microbial strain comprising deletion of a region consisting of a number of nucleotides that is preferably at least 50%, more preferably at least 60%, more preferably at least 70%, more preferably at least 80%, more preferably at least 90%, and the most preferably 100% of the total number of nucleotides constituting at least a part of the coding region of the amino acid sequence and/or the expression regulatory sequence of the gene to be disrupted of genome DNA, such as the coding region and/or the expression regulatory sequence. It is particularly preferable that the microbial strain in which a region from the start codon to the stop codon of the gene to be disrupted in genome DNA is disrupted.

Other examples of disruption of the gene to be disrupted to lower the protein activity include damage of the gene to be disrupted, such as introduction of amino acid substitution (missense mutation), introduction of a stop codon (nonsense mutation), and introduction of frameshift mutation via addition or deletion of 1 or 2 nucleotides into the amino acid sequence coding region of the gene to be disrupted in genome DNA.

Disruption of the gene to be disrupted to lower the protein activity can be achieved by, for example, insertion of another sequence into the expression regulatory sequence or the amino acid sequence coding region of the gene to be disrupted in genome DNA. While another sequence may be inserted into any region of the gene, the gene can be inactivated with certainty via insertion of a longer sequence. It is preferable that reading frames of upstream and downstream sequences of the site of insertion be inconsistent. While "another sequence" is not particularly limited as long as functions of the protein to be encoded are lowered or quenched, examples thereof include a marker gene, a gene useful for production of a target substance (a cyclic lipopeptide), and expression regulatory sequences of such genes.

Disruption of the gene to be disrupted in genome DNA can be achieved by, for example, preparing an inactive gene by modifying the gene to be disrupted so as not to produce a protein that normally functions, transforming the host strain with recombinant DNA containing the inactive gene, and causing homologous recombination between the inactive gene and a gene in genome DNA to substitute the gene in genome DNA with the inactive gene. In such a case, a marker gene may be incorporated into recombinant DNA in accordance with traits of hosts, such as auxotrophic properties. Thus, a procedure of interest is easily performed. The recombinant DNA may be linearized via cleavage with restriction enzymes, so that a strain comprising recombinant DNA integrated into genome DNA can be efficiently obtained. If a protein encoded by the inactive gene is generated, a conformation thereof would be different from that of a wild-type protein, and functions thereof would be lowered or missing.

For example, microorganisms may be transformed with linear DNA comprising an arbitrary sequence and, at both ends of the arbitrary sequence, upstream and downstream sequences of the target site of substitution (typically a part of or the entire gene to be disrupted) in genome DNA or linear DNA comprising upstream and downstream sequences of the target site of substitution in genome DNA directly ligated to each other to cause homologous recombination in regions upstream and downstream of the target site of substitution in genome DNA of the host strain. Thus, the target site of substitution can be substituted with the sequence of the linear DNA in a single step. The arbitrary sequence may comprise, for example, a marker gene sequence. A marker gene may be removed later, according to need. When a marker gene is to be removed, sequences for homologous recombination may be added to both ends of the marker gene, so as to efficiently remove the marker gene.

Whether or not the gene to be disrupted has been disrupted in the microbial strain can be verified based on a lowering in the activity of the protein encoded by the gene to be disrupted. A lowering in the protein activity can be verified by assaying the activity of the protein.

A lowering in the transcription level of the gene to be disrupted can be verified by comparing the amount of mRNA transcribed from the gene of interest with the amount of mRNA of the host strain. The amount of mRNA can be evaluated by, for example, Northern hybridization or RT-PCR (e.g., Molecular cloning, Cold Spring Harbor Laboratory Press, Cold spring Harbor, U.S.A., 2001). It is preferable that the amount of mRNA be lowered to, for example, 50% or lower, 20% or lower, 10% or lower, 5% or lower, or 0% of the amount of mRNA in the host strain.

A lowering in the amount of a protein encoded by the gene to be disrupted can be verified via Western blotting using an antibody (Molecular cloning, Cold Spring Harbor Laboratory Press, Cold spring Harbor, U.S.A., 2001). In the microbial strain according to one or more embodiments of the present invention, the amount of the protein encoded by the gene to be disrupted is preferably lowered to, for example, 50% or lower, 20% or lower, 10% or lower, 5% or lower, or 0% of the amount of the protein in the host strain.

### < Method for producing the cyclic lipopeptide according to the present invention>

Further one or more embodiments of the present invention relate to a method for producing a cyclic lipopeptide comprising culturing the microbial strain according to one or more embodiments of the present invention.

In the present invention, examples of cyclic lipopeptides include a surfactin-family cyclic lipopeptide, an iturin-family cyclic lipopeptide, and a fengycin-family cyclic lipopeptide.

A surfactin-family cyclic lipopeptide is a cyclic lipopeptide composed of 7 amino acids bound to β-hydroxy fatty acid with a chain length ranging from C1 1 to C17. Examples thereof include surfactin, esperin, lichenysin, and pumilacidin.

An iturin-family cyclic lipopeptide is a cyclic lipopeptide composed of 7 amino acids bound to β-amino fatty acid with a chain length ranging from C12 to C18. Examples thereof include iturin A, iturin A1, iturin C, bacillomycin D, bacillomycin F, bacillomycin L, bacillomycin LC (baciliopeptin), and mycosubtilin.

A fengycin-family cyclic lipopeptide is a cyclic lipopeptide composed of 10 amino acids bound to β-hydroxy fatty acid with a chain length ranging from C14 to C21. Examples thereof include fengycin A, fengycin B, plipastatin A, and plipastatin B.

According to the method of the present invention, the target cyclic lipopeptide may be produced by inoculating the microbial strain in a medium containing carbon sources, nitrogen sources, and other essential components assimilable by the microbial strain, performing culture in accordance with a conventional microbial culture method, and, after the completion of culture, purifying the target cyclic lipopeptide.

A person skilled in the art can adequately select the composition of the medium used for culture and the culture conditions in accordance with a type of a microbial strain to be used and other conditions. For example, a synthetic or natural medium may be used, provided that such medium contains nutrients necessary for the growth of the microbial strain and the biosynthesis of the target substance, such as carbon sources, nitrogen sources, inorganic acids, and vitamins, assimilable by the microbial strain used in the present invention.

Examples of carbon sources include saccharides, such as glucose, maltose, fructose, sucrose, hydrolyzed starch, and molasses; alcohols, such as ethanol and glycerol; organic acids, such as acetic acids; and lipids, such as plant oil, animal oil, and fatty acid. Any of such carbon sources can be used by itself or in combinations of two or more, and glucose or maltose is preferable.

Examples of nitrogen sources include ammonium salts, such as ammonium nitrate, ammonium sulfate, ammonium chloride, and ammonium acetate; nitrogen compounds, such as ammonia, sodium nitrate, potassium nitrate, sodium glutamate, urea, various amino acids, and amine, and natural nitrogen sources, such as peptone, yeast extract, meat extract, soybean hydrolysate, and ground beans or an extract thereof. Any of such nitrogen sources can be used by itself or in combinations of two or more, and it is preferable that ground beans or an extract thereof be used in combination with other nitrogen sources. Examples of beans that can be used include soybeans, *azuki* beans, peas, horse beans, chickpeas, lentil beans, and green beans, with soybeans being preferable.

Examples of inorganic salts include cations or anions, such as potassium ions, sodium ions, magnesium ions, iron ions, manganese ions, calcium ions, zinc ions, cobalt ions, nickel ions, copper ions, molybdenum ions, phosphoric acid ions, sulfuric acid ions, chloride ions, and nitric acid ions.

Examples of vitamins include biotin and thiamine. In addition, substances required by the microbial strain according to one or more embodiments of the present invention to grow (e.g., a required amino acid for an amino acid-requiring microbial strain) can be added, according to need.

It is preferable that culture be performed under aerobic conditions, such as shake culture or aeration agitation culture. In case of foaming, a common antifoaming agent can be used. Culture is performed at 20°C to 50°C, preferably at 20°C to 42°C, and more preferably at 23°C to 38°C. At the time of culture, a pH level is 5 to 9, and preferably 6 to 8. A culture duration is for 3 hours to 5 days, and preferably for 5 hours to 3 days.

In a preferable embodiment of the present invention, cyclic lipopeptide production using bacteria of the genus *Bacillus* is performed by adopting the composition of the medium used for culture and the culture conditions described in JP Patent No. 3,635,638. Specifically, culture is preferably performed with the use of a medium containing soy powder or an extract thereof. The term "soy powder or an extract thereof' refers to, for example, coarse-grained soy powder prepared by grinding soybeans or defatted soybeans to granular form, ground soy powder prepared by grinding soybeans or defatted soybeans to a powder form, an extract thereof (e.g., a hot water extract), or a hydrolysate (e.g., an acid hydrolysate or an enzyme hydrolysate). While the concentration of soy powder or an extract thereof is not particularly limited, the amount of cyclic lipopeptide production is increased in proportion to the concentration of soy powder or an extract thereof in a medium. In order to achieve a sufficient amount of production, accordingly, the concentration is preferably 0.5 w/w% or higher. However, soy powder or an extract thereof may not be sufficiently sterilized at high concentration. Thus, it is preferable that the concentration thereof be not more than 20 w/w%. Accordingly, the concentration of soy powder or an extract thereof is 0.5 to 20 w/w%, preferably 2 to 15 w/w%, and more preferably 4 to 12 w/w%, to achieve a greater amount of production.

The medium can be supplemented with, in addition to soy powder or an extract thereof, common assimilable carbon sources, nitrogen sources, inorganic salts, or the like. According to need, the medium can further be supplemented with, for example, amino acids and/or vitamins.

As carbon sources, glucose, maltose, sucrose, hydrolyzed starch, molasses, potato extract, malt, peat, plant oil, corn steep liquor, fructose, syrup, liquid sugar, invert sugar, alcohol, organic acid, organic acid salt, alkane, or other common carbon sources can be used. Any of such carbon sources can be used by itself or in combinations of two or more, and glucose or maltose is preferable. The carbon sources can be used at the concentration of generally about 0.01 to 50 w/w%, and preferably about 1 to 40 w/w%.

As nitrogen sources, ammonium salts, such as ammonium nitrate, ammonium sulfate, ammonium chloride, ammonium acetate, ammonium carbonate, or ammonium bicarbonate, and inorganic or organic nitrogen sources, such as ammonia, sodium nitrate, potassium nitrate, sodium glutamate, urea, peptone, meat extract, corn steep liquor, casein hydrolysate, feather meal, or yeast extract, can be used. Any of such nitrogen sources can be used by itself or in combinations of two or more, and yeast extract is preferable from the viewpoint of cyclic lipopeptide productivity. The nitrogen sources can be used at the concentration of generally about 0.01 to 30 w/w%, and preferably about 0.1 to 10 w/w%.

As inorganic salts, cations or anions, such as potassium ions, sodium ions, magnesium ions, iron ions, manganese ions, calcium ions, zinc ions, cobalt ions, nickel ions, copper ions, molybdenum ions, phosphoric acid ions, sulfuric acid ions, chloride ions, or nitric acid ions, are preferably added. While the concentration of inorganic salts to be added varies depending on culture conditions, in general, phosphate is added at about 0.01 to 5 w/w%, magnesium salt is added at about 10 ppm to 2 w/w%, and other salts are added at about 0.1 ppm to 1,000 ppm.

Examples of amino acids include L-glycine, L-alanine, L-valine, L-leucine, L-isoleucine, L-serine, L-threonine, L-phenylalanine, L-tyrosine, L-cysteine, cystine, L-methionine, L-tryptophan, L-histidine, L-proline, L-aspartic acid, L-asparagine, L-glutamic acid, L-glutamine, L-arginine, L-lysine, D-valine, and D-isoleucine, and at least one of such amino acids can be added. In the present invention, in particular, L-arginine and/or L-tryptophan is preferably added. The concentration of amino acids to be added is generally about 0.001 to 5 w/w%, and preferably about 0.01 to 1 w/w%.

Examples of vitamins include biotin, thiamine, riboflavin, pyridoxine, nicotinic acid, nicotinic acid amide, pantothenic acid, pyridoxal, pyridoxine, myo-inositol, choline, folic acid, cobalamin, and cyanocobalamin, and at least one of such vitamins can be added. The concentration of vitamins to be added is generally 0.1 to 100 ppm, and preferably 1 to 50 ppm.

Culture is performed by adding the medium to a container, such as a test tube, flask, or fermenter, with vigorous aeration. When culture is performed with the use of a container, such as a test tube or flask, aeration is performed with vigorous shaking, and the initial pH level of the medium is adjusted to 6.5 to 8.0. When high-concentration production is intended with the use of a container, such as fermenter, culture is performed by introducing sterile air with agitation. When it is difficult to perform culture because of foaming, a common antifoaming agent can be added.

A pH level of a medium is maintained at 6 to 9, preferably at 6.5 to 8.0, and more preferably at 6.9 to 7.5. A pH level is adjusted with the addition of, for example, a basic aqueous solution, such as an aqueous solution of ammonia, potassium hydroxide, sodium hydroxide, sodium carbonate, or potassium carbonate, and use of sodium hydroxide or aqueous ammonia is particularly preferable. Sodium hydroxide may be added at the concentration of about 20 w/w%. Aqueous ammonia may be added at the concentration of about 8 to 25 w/w%. Culture temperature is 25°C to 42°C, preferably 28°C to 40°C, and more preferably 30°C to 37°C.

After the microbial strain of the present invention was cultured in the manner described above, the cyclic lipopeptide accumulated in the culture product can be collected in accordance with a conventional purification method. After the completion of culture, for example, bacteria or solids are removed from the culture product via centrifugation, and the cyclic lipopeptide can be collected via ion exchange, concentration, or crystal fractionation.

### Examples

Hereafter, the present invention is described in greater detail with reference to the examples, although the present invention is not limited to these examples.

Genetic engineering described below can be performed with reference to Molecular Cloning (Cold Spring Harbor Laboratory Press, 1989). Enzymes, and cloning hosts, or the like used for genetic engineering may be purchased from commercial providers and used in accordance with the instructions. The enzymes are not particularly limited, provided that they can be used for genetic engineering.

### (Production Example 1) Preparation ofBL002 strain

The BL002 strain serving as a host strain of a cyclic lipopeptide-producing strain was prepared. In order to provide the *Bacillus subtilis* 168 strain (hereafter, it may be referred to as the " 168 strain") with the cyclic lipopeptide-producing ability, specifically, a strain comprising lpa-14 (J. Ferment. Bioeng., 76: 6, 445-450, 1993) encoding 4-phosphopantetheinyl transferase introduced into the chromosome was prepared.

First, a DNA fragment for introducing lpa-14 into the chromosome of the 168 strain was prepared. PCR was carried out using synthetic oligo DNA, and a DNA fragment comprising a scaffold for homologous recombination to be introduced into the sfp gene locus in the chromosome, lpa-14, and a chloramphenicol resistance cassette was obtained (SEQ ID NO: 5: sfpUD-lpa14-cat).

Subsequently, the DNA fragment as shown in SEQ ID NO: 5 was used to transform the 168 strain as a parent strain by the CI/CII method (*"Biseibutsu Iden-gaku Jikkenho, Iden-gaku Jikken Koza* 3" (Microbial genetic experimentation, Genetic Experimentation Course 3), Kyoritsu Shuppan Co., Ltd.). Thereafter, the resulting strains were applied to an LB agar plate containing chloramphenicol at 7.5 µg/ml and cultured at 37°C to obtain transformants. The resulting transformants were analyzed by PCR and using a DNA sequencer to isolate a strain comprising lpa-14 and the chloramphenicol resistance cassette inserted in the sfp gene locus in the chromosome of the 168 strain. This strain was designated as the BL002 strain.

### (Production Example 2) Preparation of BL002 gbsA::spec strain

First, a DNA fragment used to insert an spectinomycin resistance cassette into the gbsA gene locus was prepared. PCR was carried out using synthetic oligo DNA, and a DNA fragment comprising the upstream sequence and the downstream sequence of the gbsA gene and the spectinomycin resistance cassette was obtained (SEQ ID NO: 6: gbsAUD-spec).

Subsequently, the DNA fragment as shown in SEQ ID NO: 6 was used to transform the BL002 strain prepared in Production Example 1 as a parent strain in the same manner as in Production Example 1, except that the transformed strains were applied to an LB agar plate containing chloramphenicol at 5 µg/ml and spectinomycin dihydrochloride pentahydrate at 150 µg/ml. A strain in which a region from the start codon to the stop codon of the the gbsA gene and an intergenic region between the gbsA gene and the gbsB gene located downstream of the gbsA gene in the chromosome are deleted and the spectinomycin resistance cassette is inserted therein was isolated by analysis using PCR and a DNA sequencer from among the transformants. This strain was designated as the BL002 gbsA::spec strain.

### (Production Example 3) Preparation of BL002ΔgbsA strain

First, a DNA fragment used to remove the spectinomycin resistance cassette from the BL002 gbsA:: spec strain was prepared. PCR was carried out using synthetic oligo DNA, and a DNA fragment comprising the upstream sequence and the downstream sequence of the gbsA gene was obtained (SEQ ID NO: 7: gbsAUD).

Subsequently, the DNA fragment as shown in SEQ ID NO: 7 was used to transform the BL002 gbsA::spec strain prepared in Production Example 2 as a parent strain in the same manner as in Production Example 1, except that the transformed strains were applied to an LB agar plate containing chloramphenicol at 5 µg/ml. The resulting colonies were replicated on an LB agar plate containing chloramphenicol at 5 µg/ml and an LB agar plate containing chloramphenicol at 5 µg/ml and spectinomycin dihydrochloride pentahydrate at 100 µg/ml, and transformants exhibiting spectinomycin sensitivity were selected. A strain in which a region from the start codon to the stop codon of the gbsA gene and an intergenic region between the gbsA gene and the gbsB gene located downstream of the gbsA gene in the chromosome are deleted was isolated by analysis using PCR and a DNA sequencer from among the transformants. This gene-disrupted strain was designated as the BL002ΔgbsA strain.

### (Production Example 4) Preparation of BL002ΔgbsB strain

First, a DNA fragment used to disrupt the gbsB gene was prepared. PCR was carried out using synthetic oligo DNA, and a DNA fragment comprising the upstream sequence and the downstream sequence of the gbsB gene and the spectinomycin resistance cassette was obtained (SEQ ID NO: 8: gbsBUD-spec).

With the use of the BL002 strain prepared in Production Example 1 as a parent strain and the DNA fragment as shown in SEQ ID NO: 8, a strain in which a region from the start codon to the stop codon of the gbsB gene in the chromosome is deleted and the spectinomycin resistance cassette is inserted therein was isolated in the same manner as in Production Example 2. This gene-disrupted strain was designated as the BL002ΔgbsB strain.

### (Production Example 5) Preparation of BL002ΔgbsAB strain

First, a DNA fragment used to disrupt the gbsA gene and the gbsB gene was prepared. PCR was carried out using synthetic oligo DNA, and a DNA fragment comprising the upstream sequence of the gbsA gene, the downstream sequence of the gbsB gene, and the spectinomycin resistance cassette was obtained (SEQ ID NO: 9: gbsAU-spec-gbsBD).

With the use of the BL002 strain prepared in Production Example 1 as a parent strain and the DNA fragment as shown in SEQ ID NO: 9, a strain in which a region from the start codon of the gbsA gene to the stop codon of the gbsB gene in the chromosome is deleted and the spectinomycin resistance cassette is inserted therein was isolated in the same manner as in Production Example 2. This gene-disrupted strain was designated as the BL002ΔgbsAB strain.

### (Example 1) Production of surfactin using BL002ΔgbsA strain

The BL002ΔgbsA strain obtained in Production Example 3 was cultured under the conditions described below to produce surfactin.

First, the BL002ΔgbsA strain was inoculated in 3 ml of LB medium containing chloramphenicol at 5 µg/ml and subjected to shake culture at 37°C and 300 rpm for 16 hours. The culture solution was inoculated in 2.5 ml of a production medium (40 g/l soybean flour, 5g/l dipotassium hydrogenphosphate, 0. 5g/l magnesium sulfate heptahydrate, 0.18 g/l calcium chloride dihydrate, 0.025 g/l iron sulfate heptahydrate, 0.022 g/l manganese chloride tetrahydrate, and 30 g/l maltose monohydrate) to adjust OD 600 to 0.1 and subjected to shake culture at 35°C and 300 rpm for 72 hours. The surfactin concentration in the resulting culture solution was analyzed by HPLC under the conditions described below.

### [HPLC conditions]

Sample amount: 20 µl
Column: ODS-2, 4.6 mm × 250 mm, GL Sciences Inc.
Column temperature: 40°C
Eluate: 80 v/v% acetonitrile, 3.8 mM trifluoroacetic acid
Flow rate: 1.5 ml/min
Detector: UV detector
Wavelength: 205 nm

The surfactin concentration was quantified by preparing a calibration curve using a surfactin standard sample (Sigma Aldrich).

### (Example 2) Production of surfactin using BL002ΔgbsB strain

The BL002ΔgbsB strain obtained in Production Example 4 was cultured under the same conditions as in Example 1 to produce surfactin, except for the use of an LB medium for pre-culture, which contains chloramphenicol at 5 µg/ml and spectinomycin dihydrochloride pentahydrate at 100 µg/ml. The surfactin concentration in the resulting culture solution was analyzed in the same manner as in Example 1.

### (Example 3) Production of surfactin using BL002ΔgbsAB strain

The BL002ΔgbsAB strain obtained in Production Example 5 was cultured under the same conditions as in Example 2 to produce surfactin. The surfactin concentration in the culture solution was analyzed in the same manner as in Example 1.

### (Comparative Example 1) Production of surfactin using BL002 strain

The BL002 strain obtained in Production Example 1 was cultured under the same conditions as in Example 1 to produce surfactin. The surfactin concentration in the culture solution was analyzed in the same manner as in Example 1.

Table 1 shows the results of analysis of surfactin concentrations in Examples 1 to 3 and Comparative Example 1.

**[Table 1]**

| | Strain | Amount of surfactin production (g/l) |
|---|---|---|
| Example 1 | BL002ΔgbsA strain | 5.93 |
| Example 2 | BL002ΔgbsB strain | 6.00 |
| Example 3 | BL002ΔgbsAB strain | 5.79 |
| Comparative Example 1 | BL002 strain | 4.96 |

The results of Examples 1 to 3 and Comparative Example 1 shown in Table 1 demonstrate that disruption of either or both the gbsA gene and the gbsB gene would increase the amount of surfactin production to a significant extent. The results demonstrate that, in cyclic lipopeptide production via microbial culture, disruption of at least either one of the gene encoding betaine aldehyde dehydrogenase and the gene encoding choline dehydrogenase is effective to improve cyclic lipopeptide productivity.

### Industrial Applicability

The present invention can be used in the field of cyclic lipopeptide production.

All publications, patents, and patent applications cited herein are incorporated herein by reference in their entirety.

## Claims

1. A cyclic lipopeptide-producing microbial strain, having disruption of at least either one of the gene (1) or (2):
(1) a gene encoding betaine aldehyde dehydrogenase (EC: 1.2.1.8); or
(2) a gene encoding choline dehydrogenase (EC: 1.1.1.1).

2. The microbial strain according to claim 1, which is a genetically modified microbial strain whose host is a bacterium.

3. The microbial strain according to claim 2, wherein the bacterium is a Gram-positive bacterium.

4. The microbial strain according to claim 3, wherein the Gram-positive bacterium is a bacterium of the genus *Bacillus.*

5. The microbial strain according to claim 4, wherein the bacterium of the genus *Bacillus* is *Bacillus subtilis.*

6. The microbial strain according to any one of claims 1 to 5, wherein the cyclic lipopeptide is at least one cyclic lipopeptide selected from among a surfactin-family cyclic lipopeptide, an iturin-family cyclic lipopeptide, and a fengycin-family cyclic lipopeptide.

7. The microbial strain according to any one of claims 1 to 6, wherein the cyclic lipopeptide is surfactin.

8. A method for producing a cyclic lipopeptide comprising culturing the microbial strain according to any one of claims 1 to 7 in a medium.

9. The method for producing a cyclic lipopeptide according to claim 8, wherein the medium contains grounded beans or an extract thereof.

10. The method for producing a cyclic lipopeptide according to claim 9, wherein the beans are soybeans.
